# EUROPEAN PATENT APPLICATION

(11) **EP 2 145 623 A1**
(43) Date of publication of application: **20.01.2010**
(21) Application number: 07793985.8
(22) Date of filing: 29.01.2007
(51) Int. Cl.: A61K 31/66, A61K 38/03, A61P 43/00, A61P 39/06

(54) **PHARMACEUTICAL AND COSMETIC COMPOSITIONS FOR ACCELERATED HEALING OF WOUNDS AND OTHER SURFACE DAMAGES**

(71) Applicant: Limited Liability Company "Mitotechnology", Moscow 119180 (RU)
(72) Inventor: SKULACHEV, Vladimir Petrovich, Moscow, 119234 (RU); SKULACHEV, Maxim Vladimirovich, Moscow, 119234 (RU)
(74) Representative: HOFFMANN EITLE
(86) International application number: PCT/RU2007/000043
(87) International publication number: WO 2008/094061

(57) **Abstract**

The present Invention relates to biology and medicine, in particular, it can be used in medicine for preparing a pharmaceutical composition used for accelerated healing of wounds and the other damages by means of targeted (addressed) delivery of biologically active substances into mitochondria, performed due to electrochemical potential of hydrogen ions contained therein. The said invention can be also used for producing a composition useful in transplantation surgery for preserving transplantation material and for inhibiting rejection. In addition the invention can be used for producing cosmetic agent for improving state of the skin and for revitalization and rejuvenation thereof.

## Description

### Field of the Invention

The present invention relates to biology and medicine and specifically may be used in medicine for preparing a pharmaceutical composition that accelerates healing of wounds using the addressed (targeted) delivery of biologically active substances into mitochondria, performed due to electrochemical potential of hydrogen ions in mitochondrion. In addition the invention relates to a method of acting upon organism by targeted delivery of the required biologically active substances into mitochondria.

### Background of the Invention

Local healing of wounds under dressings is one of the main methods for first-medical-aid dressing and combination therapy of wounds, and in a number of cases it is virtually the only method (if the patients have associated diseases, extensive damages and contraindications to surgical treatment etc.).

The development of biologically active dressings has played a significant role in progress of modem methodology for topical treatment. Biologically active dressings are dressings or wound coatings on the basis of natural, artificial or synthetic materials, containing drugs and biologically active preparations, or having an active effect on wound tissues and the course of the wound process at the expense of specific properties of the basis. The basic research in the field of pathogenesis of wound process showed that no one drug can be universal and unambiguously effective in treatment of wounds of various etiology at definite stages of healing thereof.

Based on studying biological mechanisms of wound healing it was stated that treatment policy and drugs should be determined by the phase of wound process.

Three phases of wound process can be identified according to classification of wound process:
- the first phase is a phase of inflammation characterized by edema, excessive discharge, microbial invasion, development of necrosis, disorders of microcirculation and metabolic disorders;
- the second phase is a phase of regeneration, formation and maturation of granulation tissue;
- the third phase is a phase of epithelialization and reorganization of the scar.

The modem methodology of topical healing of wounds is being constructed taking into account the specific features of each phase as well as the concept based on the necessity of targeted and differentiated action of wound dressings on the wound process. In accordance with this, the basis of the above methodology is formed by the principles specified differentiated choice of dressings ensuring the predetermined effect on the wound process. In this case it is necessary that a practicing surgeon should have a choice of wound dressings and the information concerning their specific action on a wound process.

The dressings for treatment of wounds in the 1-st phase, i.e. the inflammation phase, should satisfy the following requirements: they should have sorption capacity, ensure efflux of wound discharge and microflora from the wound funds, exhibit anti-inflammatory, anti-edema, antimicrobial, and protcolytic activities, and facilitate normalization of microcirculation and regeneration processes.

In the 2-d phase of a wound process, the dressings should provide conditions for normal course of proliferation process for cells of regeneration tissue, beneficially effect on angiogenesis, mobility and synthesis of epithelial cells, suppress vegetative microflora in the wound and protect the wound from secondary infections.

In the 3-d phase of a wound process, the dressings should provide conditions for normal course of proliferation process for cells of regeneration tissue and epithelium, beneficially effect on angiogenesis, mobility and synthesis of epithelial cells, suppress vegetative microflora in the wound and protect the wound from secondary infections, and create conditions for prevention from formation of keloid scars.

In most cases modern wounds are characterized by failure of healing due to various reasons connected both with attenuation of immunological status and associated diseases. This pathology is characterized by a sustained and prolonged wound process, liability to recurrences, and often leads to total or partial disability. The category of such patients is becoming a serious medical and social problem all over the world. For treatment of such patients a complex of biologically active dressings including those that stimulate wound-healing process have been created.

The development of wound coatings having antioxidant activity that make it possible to stop in flammation at early stages of wound healing and create conditions for normal regeneration process plays an important role in creating a spectrum of biologically active dressings. However, the use of conventional antioxidants is connected with certain difficulties associated with non-targeted localization of these substances in a cell and the whole organism.

### Essence of the invention

The present invention is based on the principle of concentrating biologically active substances in the mitochondria of a living cell via the use of the energy of the electrochemical potential of hydrogen ions and Skulachev ions. Such an approach has unexpectedly made it possible to achieve a multifold decrease in the dosage of the employed biologically active substances, the targeted effective influence on mitochondria, which arc the key element in the most important intracellular processes, this provides the opportunity for a multifold decrease of the probability and strength of unfavorable side effects.

Thus, one of the aspects of the present invention is to provide a method of acting on an organism via targeted delivery of biologically active substances to mitochondria at the expense of the energy of the electrochemical potential of hydrogen ions.

The general chemical structure of such compounds is represented by the following formula (I): 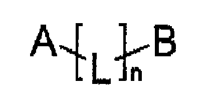 wherein A is the effector group which is an antioxidant 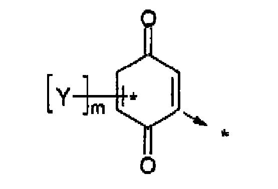 and/or a reduced form thereof wherein m is an integer from 1 to 3; each Y represent the same or different substituents which are a lower alkyl or a lower alkoxy; or two vicinal Y are interconnected so as to form the structure: 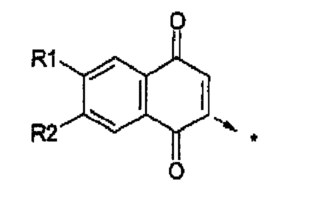 and/or a reduced form thereof
wherein R1 and R2 are the same or different and independently represent a lower alkyl or a lower alkoxy;
L - is a linker group that is a) a straight or branched hydrocarbon chain optionally substituted with one or more substituents and optionally containing one or more double or triple bonds if necessary;
b) a naturally occurring isoprene chain;
n is an integer from 1 to 20;
B is a) a Skulachev ion Sk;
Sk⁺ Z⁻
wherein Sk is a lipophilic cation;
Z is a pharmacologically acceptable anion;
b) a charged hydrophobic peptide containing 1-20 amino acids,
with the proviso that in the compounds of structure (I) Λ is neither ubiquinone (e.g. 2- methyl-4,5-dimethoxy-3,6-dioxo-1,4-cyclohexadienyl) nor tocopherol or mimetic of superoxide dismutase or ehselen; wherein L is a divalent decyl or divalent pentyl or divalent propyl radical; and B is triphenylphosphonium; as well as solvates, isomers, pharmaceutically acceptable salts thereof.

Another aspect of the present invention is a pharmaceutical composition suitable for targeted delivery of a biologically active substance into cell mitohondria comprising a compound having structure (II), wherein A represents plastoquinone moiety of the formula: 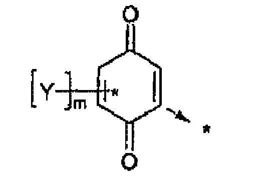 Y is methyl, m is 2;
L - is a linker group that is a) a straight or branched hydrocarbon chain optionally substituted with one or more substituents and optionally containing one or more double or triple bonds if necessary;
b) a naturally occurring isoprene chain;
n is an integer from 1 to 20;
B is a) a Skulachev ion Sk;
SK⁺ Z⁻
wherein Sk is a lipophilic cation;
Z is a pharmacologically acceptable anion:
b) a charged hydrophobic peptide containing 1-20 amino acids,
with the proviso that in the compounds of structure (I) A is neither ubiquinone (e.g, 2- methyl-4,5-dimethoxy-3,6-dioxo-1,4-cyclobexadienyl) nor tocopherol or mimetic of superoxide dismutase or ebselen; wherein L is a divalent decyl or divalent pentyl or divalent propyl radical; and B is triphenylphosphonium;
as well as solvates, isomers, pharmceutically acceptable salts thereof.

Another aspect of the present invention is a pharmaceutical composition for accelerating healing of wounds and other surface damages using mitochondrlay-targeted antioxidants, comprising a compound of the structure (I) - SkQ1 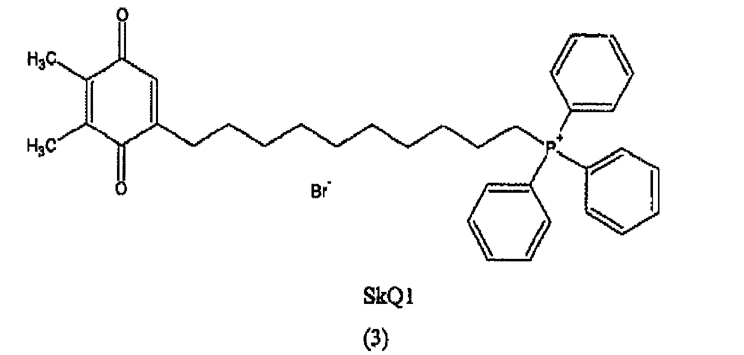 or a similar compound (to which SkQ1, SkQR1 and other mitochondrially-tatgeted antioxidants arc related).
In connection with this aspect of the invention, there is proposed;
- use of mitochondrially-targeted antioxidants for overcoming consequences of burns and promoting healing of wounds and surgical sutures;
- use of mitochondrially-targeted antioxidants to promote healing of ulcers including trophic ulcers that include diabetic wounds and ulcers;
- use of mitochondrially-targeted antioxidants as anti-inflammatory drugs;
- use of mitochondrially-targeted antioxidants during surgical operations to protect healthy tissues from damages;
- use of mitochondrially-targeted antioxidants in transplantology for suppressing rejection of tissues and for preserving the transplantation material;

The pharmaceutical composition accelerating healing of wounds and other surface damages comprises therapeutically or prophylactically reasonable amount of a compound of structure (I), and at least one pharmaceutically acceptable diluent or carrier. The pharmaceutically acceptable diluent or carrier may be filler or attenuant, or a mixture thereof. The term "therapeutically reasonable" means the amount of compound (I) that results in the desired biological or medicinal response in a patient who receives treatment by a physician or a veterinarian. The term "prophylactically reasonable amount" of a substance means the amount of a compound of structure (I), that prevents or suppress a disease, or alleviates the course of a disease in a patient suffering from medical condition which a physician or a veterinarian tries to prevent, suppress or alleviate.

In one of the aspects of the present invention, the patient is a human,

"Wounds and other surface damages" include, but are not limited to, wounds associated with damages of skin epithelium, epithelium of cornea, surfaces of gastrointestinal tract, epithelium of lungs and internal surfaces of liver vessels, blood vessels, uterus, vagina, urethra or respiratory tract. The present invention also provides therapy of wounds and other surface damages associated with long-term defects of epithelium and recurrent epithelial erosion such as surgical wounds, excision wounds, blisters, ulcers, other damages, scratch marks, erosions, avulsive wound, cults, running sores, furuncles, and thermal or chemical burns. Such wounds may be caused both by mechanical damages and as a result of other diseases such as diabetes, corneal dystrophy, uremia, food-deficiency, vitamin-deficiency, obesity, infection, immunodeficient disease, or complications associated with systematic steroid application, radiation therapy, non-steroid anti-inflammatory preparations and anti-cancer preparations.

Compounds of formula (I) can be used for acceleration of healing of wounds and other surface damages independently on the reasons that caused them.

Another aspect of the invention is a method for preparing a composition that may be used in cosmetology for improving state of skin, cutaneous rejuvenation, prophylaxis or therapy of age-related skin changes, including chronological 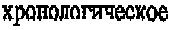 aging, photoaging, damages of skin caused sunlight or UV-radiation. A method for preparing a cosmetic composition for promoting hair growth, prevention from hair loss, and recovery of hair-covering is also an aspect of the invention.

Use of the pharmaceutical and cosmetic compositions related to the present invention, for treatment of humans and other mammals may be percutaneous, intradermal, oral, rectal, parenteral, intracisternal, intravaginal, intraperitoneal, buccal, ocular or nasal depending on severity and localization of the disease. Prescription may be both therapeutic and prophylactic. Liquid pharmaceutical compositions for oral administration include, but are not limited to, pharmaceutically acceptable emulsions, microemulsions, solutions, suspensions, syrups and elixirs. Solid pharmaceutical compositions for oral administration include, hut are not limited to, capsules, tablets, pills, powders or granules. Pharmaceutical compositions for percutaneous or intradermal administration include, but are not limited to, ointments, pastes, creams, lotions, gels, powders, solutions, sprays, inhalants or dressings. Pharmaceutical compositions for injections include, but are not limited to, sterile aqueous or oil suspensions. Pharmaceutical compositions for rectal or intravaginal administration are used preferably in the form of suppositories.

The pharmaceutical compositions for prophylactic applications can be used for therapy of focuses for possible appearance of wounds or first causes of wound surfaces such as contact lens, solutions for purifying and washing contact lens, containers for storing or transportation of contact lens, ophthalmic drops, solutions for surgical washings, ear drops, eye-bandages and cosmetic products for eye area.

Another aspect of the present invention relates to ophthalmological devices, surgical devices, audiologic devices, and articles or products, which contain pharmaceutical compositions according to the present invention (for example, gauze bandages and dressings, or plasters).

If a compound of formula (I) is administered in the form of pharmaceutical composition, the compound of formula (I) should be mixed according to the formulation with the suitable amount of a pharmaceutically suitable diluent or carrier in such a manner that provides the proper form for administration to a patient. The term "diluent" is related to a attenuant, an adjuvant, a filler or a carrier with which the compound of formula (I) is mixed for administration to a patient. Such pharmaceutical carriers may be liquids such as water and oils including petroleum, animal, vegetable, and synthetic oils such as peanut butter, soybean oil, mineral oil and the like. Pharmaceutical diluents may be physiological solution, acacia gum, gelatin, starch, talc, keratin, colloidal silver, urea *etc*. The composition may also include adjuvants, stabilizers, thickeners, lubricants and coloring agents.

Compounds and compositions related to the present invention may be administered in the form of capsules, tablets, pills, lozenges, granules, syrups, elixirs, solutions, suspensions, ointments, sprays, emulsions, suppositories, sustained-release formulations, or in any other form suitable for administration to a patient.

One of the aspects of the present invention is the use of compounds of formula (I) and the pharmaceutical compositions in the form of creams, ointments, or tablets for percutaneous or oral administration.

Another aspect of the present invention is a pharmaceutical composition, comprising one or more additional therapeutical preparations in addition to compound (I). The list of additional therapeutical preparations includes, but is not limited to, a growth factor, anti-inflammatory preparations, vasopressor preparations, collagenase inhibitors, steroids, matrix metalloprotease inhibitors, ascorbates, angiotensin II, angiotensin III, calreticulin, tetracyclines, fibronectin, collagen, thrombospondin, growth factors, keratocyle growth factors, fibroblast growth factors (including fibroblast growth factor FGF-2), insulin-like growth factors, epidermal growth factor, hepatocyte growth factor, and hyaluronic acid.

Therapeutically reasonable amount of a compound of formula (I), required for treatment of a particular disease or symptom, depends on the nature of the disease or the symptom and the method of administration, and should be determined in consultations with attending physician.

The experiments carried out showed that compounds of formula (I) may be effective in treatment of the above mentioned diseases and improving a patient's state if they are used in the composition with dosage from 0,3 picomole per 1 g of the composition to 30 micromole per 1 g of the composition depending on the particular case of the use. The effective dosage for substance SkQ1 may be from 185 picograms per I g of the composition to 185 mg per 1 g of the composition.

Examples of pharmaceutical and cosmetic compositions related to the present invention.
1. Ointment-1
Active substance (SkQ1): 15 mcg
Olive oil: 20 g
Vaselinum album: up to 100 g
2. Ma_{3b}-2)
Active substance (SkQ1): 1,5 mcg
Olive oil: 20 g
Vaselinum album: up to 100 g
3. Crcam-1 for percutaneous application
Active substance (SkQ1): 15 mcg
Olive oil: 5.0 g
Cetyl alcohol: 2.0 g
Stearinic acid: 5.0 g
Glyceryl ester of aliphatic acid 12.0 g
Tween 60: 5.0 g
Propyleneglycol: 5.0 g
Methylparaben: 0.1 g
Propylparaben: 0.02 g
Water (distilled) up to 100 g
4. Cream-2 for percutaneous application
Active substance (SkQ1): 1,5 mcg
Olive oil: 5.0 g
Cetyl alcohol: 2.0 g
Stearinic acid: 5.0 g
Glyceryl ester of aliphatic acid 12.0 g
Tween 60: 5,0 g
Propyleneglycol: 5,0 g
Methylparaben: 0.1 g
Propylparaben: 0.02 g
Water (distilled) up to 100 g
5. Suppositories
Active substance (SkQ1): 15 mcg
Witepsol^{™} W-35: 100.0 g
6. Film coating (variant 1)
Active substance (SkQ 1 ): 2 mcg
Chitin derivative polymer 100.0 g

The further aspects of the invention are the Experimental Examples given below confirming the possibility or use mitochondrially-targeted antioxidants (for instance, SkQ or SkQR1).

### Brief description of the Figures:

Fig. 1 shows the increase of the average area of human fibroblasts induced by incubation in the presence of SkQ 1.
Fig. 2 shows that incubation of human fibroblasts with SkQ1 results in appearance of cells having large area. The cell area is given in conventional units.
Fog.3 shows an increase in a number of vinculin contacts per a coll induced by preincubation of human fibroblasts in the presence of SkQ1.
Fig.4 shows the influence of various concentrations of SkQ1 (incubation for 1 day in the presence of antioxidant and 17 days without a substance) on the length of zone of human fibrablast migration into the "wound" for 1 day
Fig.5 shows the quantitative evaluation of the influence of SkQ1 on the rate of fibroblast migration into the "wound". A number of cell rows that entered the wound for 24 hours.

### Detailed Description of the Invention

### Experimental Examples

### 1. Mitochondial antioxidant SkQ1 causes differentiation of human fibroblasts into myofibroblasts.

Myofibroblasts are formed as a result of differentiation of normal fibroblasts and differ from them by the larger sizes, better adhesion to the support (*in vivo* -- to extracellular matrix, *in vitro* - to plastic), the structure of cytoskeleton and contractile activity.

Myofibroblasts play the most important role in healing of wounds (both surface and internal wounds), primarily because of their ability to contract and tighten lips of a wound.

The experiments presented below demonstrate clearly that incubation of subdermal human fibroblast with mitochondrial antioxidant SkQ1 leads to stable differentiation of essential part of cells to myofibroblasts.

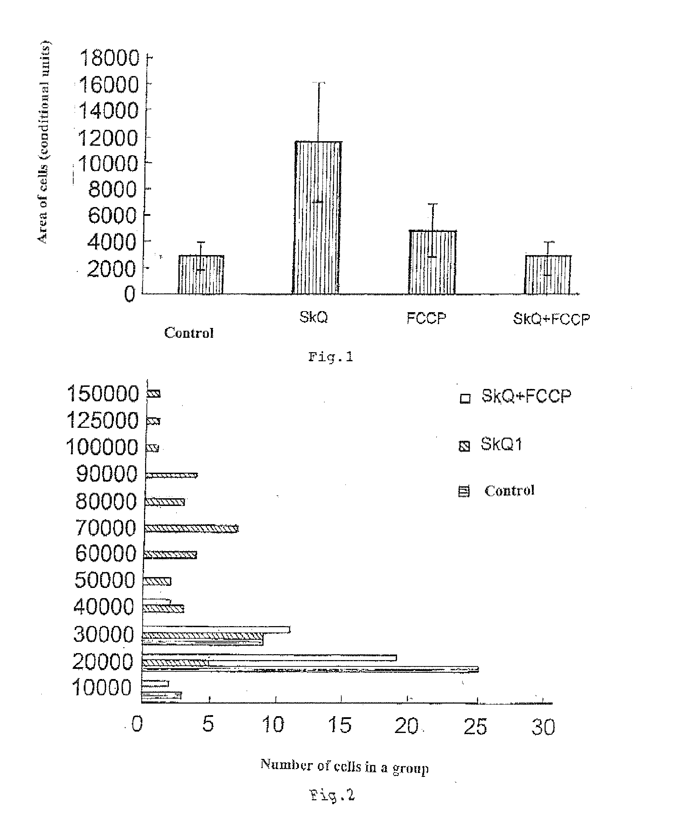

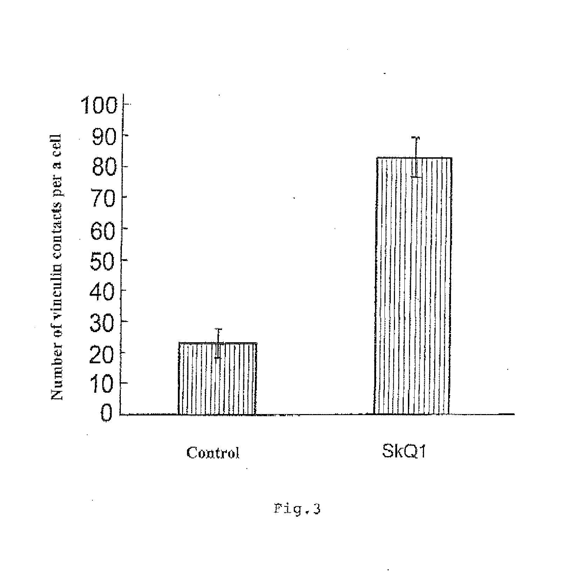

Significant changes in morphology of cells and an increase in their area were discovered in studies of subcutaneous diploid human fibroblasts treated with SkQ1 (which was added to cultural medium up to concentration 20 nM). A more sophisticated analysis showed that after treatment in most primary fibroblast cultures, division of cells in two populations was observed: in one population the area was within the limits of differences observed in control and the other population consisted of the cells whose area was 3 to 5 times larger than maximum values for control cell population. In this case an average area of cells in the culture increased compared to the control. Decoupler FCCF hinders accumulation of SkQ1 in mitochondria and prevents from changes in morphology of fibroblasts (Fig.1).

The average values of area were increased more than 2 to 3 times, but this does not also reflect the full picture of changes. The data of size distribution of cells (Fig. 2) show that a significant cell population appears in the culture whose average area is 5 to 6 times larger than the control one.

These data demonstrate clearly that morphology of the SkQ1 treated fibroblast corresponds to parameters of myofibroblasts.

The main marker for myofibroblasts is a smooth-muscle form of actin, which determines their contractile activity and is virtually absent in normal fibroblasts. Results of immunofluorescence staining on the actin smooth-muscle form show a sharp increase in content of this protein in the "gigantic" cells that appear in response to treatment with SkQ1, in comparison with the content of the smooth-muscle form of actin in normal fibroblasts.

One of the specific features of myofibroblasts is also their better adhesion to the matrix, this promotes their migration into the wound and tightening lips of the wound, in the experiments, the authors of the present invention evaluated a number of tight junctions of the cells with substrate containing a specific contact protein - vinculin. Comparison of the control cells with the cells treated with SkQ1 presented in Fag.3 demonstrates an increase in a number of contacts per a cell; this confirms an enhancement of adhesion of cells to the substrate.

The above presented data demonstrate clearly that the treatment of human fibroblasts with mitochondrially-targeted antioxidants, for example SkQ1, leads to transfomation of the essential part of fibroblasts into myofibroblasts; this should increase the rate of wound conglutination and repairing the damaged tissues in a human or an animal. It is important to note that short-term incubation (for 1 to 2 h) with SkQ1 is sufficient to induce such transformation, and further myofibroblast population is maintained in the culture for 4 to 6 weeks (or more possibly) in the absence of SkQ1.

### 2. SkQ1 causes an increase in expression of specific proteins associated with accelerated wound coaglutination.

The experiments carried out by the authors of the present invention showed that SkQ1-induced rearrangement of the cell actin skeleton with enhancing stress fibrils. This points to enhancement of contractile activity and mobility of cells. This conclusion was also confirmed by analyzing the amounts of non-muscular myosin connected with actin fibrils, This analysis was carried out using immunofluorescence (IF) staining of myosin and actin included in the stress fibrils of fibroblasts treated by SkQ1, and control cells.

Further the authors of the invention analyzed the content of the basic protein of the extracellular matrix - fibronectin - in the culture of SkQ1-treated fibroblasts. Using IF staining they showed that the content of fibronectin increases essentially both in the monolayer field and in the cell migration zone. The fibronectin matrix is formed both as a result of protein secretion by cells and by organization of the matrix components that are present in a medium as a part of a serum. The fibronectin produced by fibroblasts has definite structural distinctions that make it possible to obtain selective antibodies. The use of such antibodies allowed the authors to show that intracellular production of total fibronectin by fibroblasts increases under the auction of SkQ1, this also confirms that SkQ1 is able to accelerate healing of wounds of the various nature.

### 3. Accelerated wound conglutination on a model of human fibroblast monolayer.

A damage of a monolayer of human fibroblasts is a model of a wound *in vitro*, because fibroblasts form the basis of connective tissue whose structure is disordered in the case of any damages. It is well known that fibroblasts are ones of the first that penetrate in a wound region, and this determines the wound healing to an essential extent.

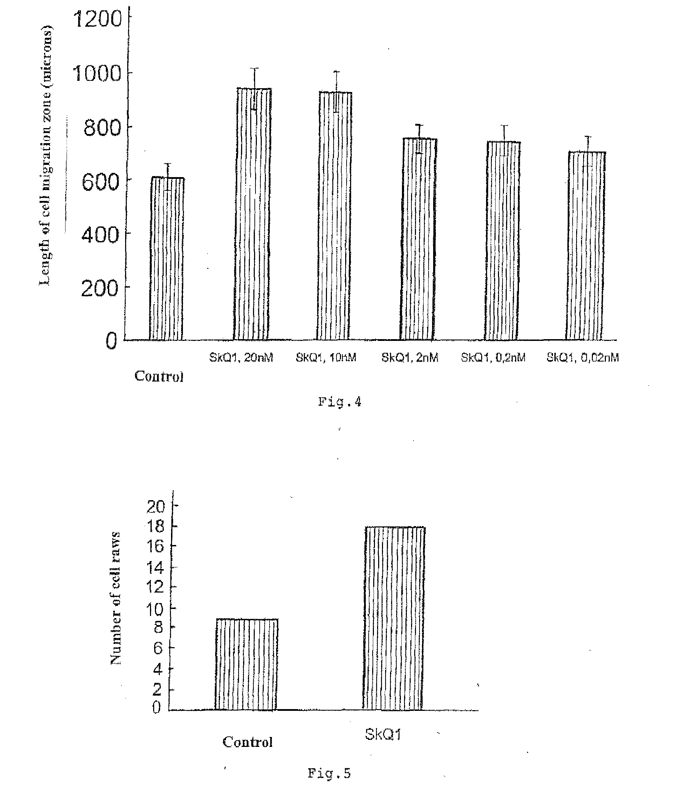

The fibroblast monolayer was damaged by a sterile scalpel as a result an expended rupture was formed in the monolayer (of the length of several millimeters) and of width about 2000 micrometers. The fibroblast capability of wound healing was evaluated by the length of the "migration zone" of cells formed within the wound for a definite period of time (24 h). The length of the migration zone directly depends on the rate of conglutinating the artificially made damage. The results of tests for several concentrations of SkQ1 are presented in Fig. 4. Like in the study of the cell morphology, it is seen that short-term incubation with SkQ1 caused an effect that was kept for a long period of time in the absence of SkQ1.

The acceleration of conglutinating the damage caused by SkQ1 is also seen from calculation of quantity of cell raws entered into the wound for 24 h (Fig. 5).

The results obtained witness in a conclusive way that mitochondrially-targeted antioxidants, for example SkQ1 are capable of stimulating accumulation of fibroblasts in a wound and transformation of them into myofibroblasts. Both these effects should promote healing of wounds of the various nature.

### 4. Acceleration of moving an epithelial layer into a wound in vitro under the action of SkQ1.

The formation of new epithelial layer on a wound surface, so called epithelialization, plays the most important role in wound-healing process, in addition to fibroblasts movement and contraction. To study the effect of SkQ1 on this process *in vitro* the culture of epithelial cells of IAR line was used. The treatment with SkQ1 did not result in essential changes in morphology of epithelium. The ability of cells to form dense "islands" and a single epithelial layer was not destroyed. The studies of expression of the specific for epithelium protein of intercellular unions - E-cadherin - confirmed preservation of the epithelial structure under the action of SkQ1. At the same time, the content of actin stress fibrils in the cells increased essentially; this pointed to a possible increase in their mobility. This effect was investigated on *in vitro* model of a wound which is similar to that used in the experiments with fibroblasts. As in the case of fibroblasts, treatment of SkQ1 essentially accelerated migration of epithelial cells into the wound. Unlike fibroblasts, the epithelial cells moved into the wound by a single layer with remaining the specific morphology and intercellular unions.

Like the experiments with fibroblasts, the effect of SkQ1 on the mobility of epithelium remained for a long period of time after withdrawal of the preparation.

The results obtained indicate that mitochondrially-targeted antioxidants, for example SkQ1 are capable of stimulating epithelialization of wounds of the various nature and healing of wounds at this stage.

### 5. Medico-experimental studies of biologically active wound coatings with immobilized mitochondrially-targeted antioxidant SkQ-1.

In this experimental example, the authors of the present invention demonstrated that the use of certain concentrations of mitochondrially-targeted antioxidants (by the example of SkQ1 immobilized as a part of the wound coating on the polymer base) makes it possible to accelerate essentially healing of wounds and healthy influences on the course of wound process.

### Results

### 1. Medicobiological description of wound healing

As the base for immobilization of the mitochondrially-targeted antioxidant SkQ-1, film wound coatings were used obtained from a complex of polyvinyl alcohol (PVA) and polysaccharide of animal origin (chitosan).

As the starting raw materials the following substances were used:
1. Chitosan isolated from crill,
2. Low-molecular polyvinyl alcohol (of medical grade) (PVA).

The formation of the wound coatings from PVA and chitosan was carried out by a casting method on a mirror metal substrate. A crosslinking agent was added to the polymer solution. Drying was performed at room temperature.

Introducing the antioxidant SkQ-1 in biosynthetic coatings was carried out at the stage of formation of polymer solutions. The introduction of biologically active ingredients into polymer complexes at the stage of their formation is technologically reasonable and effective, because it provides the minimum risk of inactivation of drugs and the best conditions for their unifom distribution in the structure. Antioxidant SkQ-1 was introduced in concentration of 0.02 meg/g of polymer.

The studies of specific activity of dressings with SkQ1 were canted out in animal experiments on white rats. Medicobiological studies of effectiveness of dressings with SkQ1 have been earned out in the experiment wherein the fanned full-thickness skin wounds with septic inflammation were treated. The experiment was performed on 20 white linear outbred rats weighing 150 to 200 g. The full-ihickness skin wounds were originally formed in interscapular region in the rats. The wounds with nonsuppurative (aseptic) inflammation were modelled in animals. To form wounds on backs of the animals (rats) after depilation, under ether anesthesia a full-thickness region of skin was exsected of the diameter up to 2 cm to subiculum fascia, and the wound was treated using the dressings of various formulations with each of them including antioxidant SkQ1. Depending on the type of dressing used for treating the wounds the animals were divided in groups:
1 - a bulky dressing (control group)
2 - a film coating with immobilized SkQ1 in amount of 0.02 meg/g of polymer (experimental group);

The course of the wound process was monitored on the basis of
1) the data of clinical observations concerning the character of the course of wound process:
   - the presence or the absence of edema of the wound lips and tissues, hyperemia, and manipulation tenderness in the wound region;
   - the presence or the absence of necrotic detritus and wound exudate on the wound surface;
   - statement of time for purifying the wounds from purulo-necrotie detritus, terms of delimitation of inflammation, appearance of granulation tissue, degree of its maturation, beginning of epithelialization.
2) the objective criteria of the course of wound process:
   - a planimetric method for evaluation of a decrease in the area of the wound defect;
   - dynamic morphological assessment of biopsy samples from wounds of the experimental animals.

Prior to treatment the initial wounds were characterized by moderate bleeding sickness and a small edema typical of primary traumatic damage. Dressing changes were made every day, After removal of the dressing, debridement of wounds was carried out using solutions of anti-infective agents. During every dressing change the biopsy samples were taken from two animals for morphological analysis.

According to the data of clinical observations, on the first day after application of dressings in the experimental group of animals wherein the treatment of wounds was carried out with polysaccharide films with SkQ 1-0,02, the minor amount of exudate was determined in the wound fundus, the wound fundus was tidy of pale-pink colour with good vascularization and without inflammation signs. The wound lips were densely fixed. The wound dressing was removed without any difficulties. Adhesion of the dressings to the wound was observed in the control group wherein the wound treatment was performed using gauze bandages, but the bandages were removed without secondary hæmorrhage. The wound fundus was pale-pink and without pronounced inflammation and edema.

On the second day in the experimental group of animals wherein the treatment of wounds was carried out with polysaccharide films with SkQ1-0.02 the wounds were moist with sites of greyish deposit on the fundus. The wound lips were densely fixed and had the features of the pronounced marginal epithelialization. The wound dressing was removed without secondary hemorrhage. The wound defect area decreased by 14.8%. In the control group, after removing the gauze bandage, the wound was slightly bled, the wound fundus had fibrin elements without exudate. The wound defect area decreased by 0.7 %.

On the third day in the experimental group, the wound fundus was palc-pink with small yellowish deposit and made of granulation tissue. The films were not lysed and virtually not swollen, they were removed without injuring the subjacent tissues. The wound defect area decreased by 20.2% in comparison with the 2-d day. In the control group, after removing the gauze bandage, the wound was slightly bled, the wound fundus had fibrin elements without exudate. The wound defect area decreased by 23.6 % in comparison with the 2-d day,

On the forth day in the experimental group wherein the treatment of wounds was carried out with polysaccharide films with SkQ1-0.02, the wound fundus was pale-pink with small yellowish deposit and made of granulation tissue without edema and inflammation signs. The films were not swollen and removed without injuring the subjacent tissues. The wound defect area decreased by 17.5% in comparison with the 3-d day. In the control group, after removing the gauze bandage, the wound remained almost unchanged in the comparison with the previous day: the wound fundus was of pale-pink colour, had fibrin elements without exudate. The wound defect area decreased by 5.6% in comparison with the 3-d day.

On the seventh day in the experimental group, the wounds essentially decreased in size, the wound fundus was dry and covered with granulation tissue of pail-pink colour, marginal epithelialization was pronounced, The wound defect area decreased by 42.4% in comparison with the 4-d day. In the control group, after removing the gauze bandage, the wound was not bled, the wound fundus contained small amount of exudate, was of pale-pink colour and had sites of grayish deposit. The wound defect area decreased by 8.9% in comparison with the forth day.

According to the results of clinical observations of the course of the wound process in the experimental wounds and effectiveness of the dressings containing immobilized SkQt, it Was ascertained that in treatment of the experimental full-thickness skin wounds, the polysaccharide films with SkQ1 in concentration 0.02 mcg/g are effective from the point of view of the positive effect on the reduction of inflammation and the dynamics of contraction, of a wound defect

### 2. Cytological and morphological investigations of SkQ1 effect on wound healing.

In parallel with the medicobiological description of the wound healing, a morphological assessment of the tissue samples from the wounds was carried out in the course of their treatment with dressings containing SkQ1 in various concetnrations.

### Methods of Investigation

Light microscopic studies of 350 semifine sections was carried out on the 1, 2, 3, 4 and 7^{th} day of the wound treatment. The 1^{st} and 2^{d} days of the wound treatment corresponded to the phase of inflammation. The 3^{d}, 4^{th} and 7^{th} days corresponded to the phase of the formation and maturation of granulation tissue. The number of fibroblasts and vessels was counted at all steps of the investigations. The counting was carried out in 30 arbitrarily selected visual fields of 100 MM² area each with 1000-fold magnification (immersion) with calculating the arithmetic mean.

### Results of investigation

On the 1^{st} day, in all groups of animals an inflammatory response pronounced to a various extent was observed. Intercellular distances were increased, this points to the development of edema. In the field of wound fundus, single fibroblasts and capillarics appeared among changed vessels of the medium and large calibre and adipocytes.

In the experimental groups of animals wherein the wounds were treated with SkQ1 in various concentration, the inflammation was not so pronounced as in the first group of animals. There was not observed marginal standing of leukocytes and paravasal tissue hydration. On the wound surface, there were fibrinous deposits with segmental leukocytes and cell debris. In the upper wound layers macrophages and small amounts of segmental leukocytes were observed. Fibroblasts and single capillaries appeared.

On the 1^{st} day of treatment, the maximum amount of fibroblasts was in the group of animals treated with dressings containing the minimum amount of SkQ1 (0.02 mcg/g) -6.93, the decrease in the number of fibroblasts is proportional to the increase of the content of the antioxidant in the film (Table 1).

On the 1^{st} day in the experimental groups of animals in the wound there were observed both young fibroblasts having large nucleus and cytoplasm poor in organelles, and functional large fibroblasts with the pronounced granular cytoplasmic reticulum, with large mitohondria and a great number of polyribosomes.

The maximum number of minute vessels were also determined in the group of animals wherein the amount of SkQ1 in the film was minimum (Table 2).

Thus, on the 1^{st} day of the treatment, the state of the wounds treated with SkQ1 was much better than that in the control group of animals. In the group of animals wherein the amount of SkQ1 (0.02 mcg/g), the number of fibroblasts and minute vessels was greater than that in other experimental groups.

**Table 1. An average number of fibroblasts in treatment of wounds with SkQ1**

| | Duration of treatment, days | | | | |
|---|---|---|---|---|---|
| | 1 | 2 | 3 | 4 | 7 |
| Control | 0.15 | 0.21 | 8.6 | 12.73 | 19.6 |
| Solution of SkQ1 -0.2 mcg/g | 3.93 | 8.93 | 9.33 | 13.13 | 16.13 |
| Polymer with SkQ1-0.2 mcg/g | 5.13 | 5.46 | 19.75 | 22.1 | 10.6 |
| Polymer with SkQ1-0.02 mcg/g | 6.93 | 5.93 | 18.06 | 15.26 | 15.3 |
| Polymer with SkQ1-2.0 mcg/g | 3.66 | 7.53 | 16.73 | 27.06 | 44.4 |

**Table 2. An average number of vessels in treatment of wounds with SkQ1**

| | Duration of treatment, days | | | | |
|---|---|---|---|---|---|
| | 1 | 2 | 3 | 4 | 7 |
| Control | 0.11 | 0.09 | 1.4 | 1.66 | 1.8 |
| Solution of SkQ1 - 0.2 mcg/g | 0.13 | 0.13 | 0.93 | 1.73 | 1.06 |
| Polymer with SkQ1 -0.2 mcg/g | 0.06 | 0.06 | 2.0 | 1.6 | 0.66 |
| Polymer with SkQ1 -0.02 mcg/g | 0.2 | 0.4 | 1.13 | 0.73 | 0.6 |
| Polymer with SkQ1 -2.0 mcg/g | 0.06 | 0.4 | 0.93 | 2.26 | 2.0 |

On the 2^{d} day in the wounds of the control animals, the inflammation signs remained: the tissue was edematic, and destructive changes propagated. Neutrophilic infiltration, migration of globocellular clements such as lymphocytes and macrophages were determined. The active processes of organization of granulation tissue were observed in the experimental groups of animals. In addition to lymphocytes and macrophages, the newly formed capillaries appeared near which the flusters of fibroblasts were observed. The number of fibroblasts more than doubled in comparison with the previous day of treatment in the group of animals wherein the maximum content of SkQ1 was used (Table 1). In the case of such treatment the number of vessels increased more than 6-fold and achieved the same values that were recorder in the case wherein minimum amounts of SkQ1 were used (Table 2).

On the 3^{d} day of the observations, the activation of proliferation processes was noted in the wounds of the experimental animals that led to a significant increase in the number of fibroblasts and vessels in the wound. The effect was observed in the groups of animals received various amounts of SkQ1 (Table 1). An increase in the number of vessels in the wound was much more significant and also observed in all groups of animals received SkQ1 (Table 2).

The ultrastructural analysis of the samples of the granulation tissue for the case of wound treatment with SkQ1 has determined fibroblasts in state of mitosis, functionally active fibroblasts with large mitochondria, the hypertrophic granular cytoplasmic reticulum that form cavities filled with finely grained contents. The spindle-shaped cells with long processes, similar to fibroblasts, and smooth muscle cells were found. The cytoplasm of such cells contained a great number of polyribosoms which were preferably localized in the conical cytoplasmic zone, and the longitudinal myofilaments between which dense bodies were scattered, similar to those in smooth muscle cells. These features made it possible to identify these cells as myofibroblasts which play the important role in contraction of wounds in healing as well as they actively synthesize extracellular matrix components. The formation of myofibroblasts subsequent to depositing the first collagen fibers in the granulation tissue suggests that a certain quantity of fibroblasts are transformed into myofibroblasts with acquiring contractile ability and influencing on the space orientation of the newly synthesized collagen fibers.

In the granulation tissue there are many capillaries formed by 2-3 endothelial cells. The basal membrane surrounding the newly formed capillaries is not pronounced. The granular cytoplasmic reticulum is presented by narrow profile, a lot of ribosoms, mitochondria. In addition to small dense mitochondria, the organelles of the increased size with the cleared matrix were found. Such ultrastructure of endothelial cells points to their high functional activity.

Thus, on 3^{d} day of the wound treatment with SkQ1 the pronounced stimulation of proliferation processes was observed that led to a significant increase in the number of fibroblasts and vessels. The functional activity of fibroblasts increased; myofibroblasts appeared.

On the 4^{th} day the granulation tissue in the control group of mammals was less mature than that in the experimental groups: a significant number of macrophages was observed, mums fibroblasts there were more young cells capable of proliferating, In the experimental groups of animals there were no pronounced changes in the wound tissues in comparison with the previous date. The granulation tissue looked mature and was presented in the main by collagen-synthesizing fibroblasts with large nuclear, the granular cytoplasmic reticulum that occupied almost whole cytoplasm, large mitochondria and a great number of polyribosomes. Horisontal orientation of fibroblasts was tracked. There were fibroblasts in state of mitosis, macrophages were single. The number of fibroblasts in all groups of animals changed to a minor extent in comparison with the previous day of treatment. The maximum increase in the number was observed in the case of maximum content of SkQ1 in the dressing (Table 1). The number of vessels in this group of animals increased about two-fold, and in the other groups it changed to a minor extent (Table 2).

On the 7^{th} day of the study, the wound defects in the control group of animals were filled with the growing granulation tissue with a great number of fibroblasts, vessels and macrophages. In the experimental groups of animals the degree of maturity of the regeneration tissue was higher and the wound defects were filled by the mature granulation tissue. In the group of animals received dressings with the maximum content of SkQ1, the young connective tissue was presented by the collagen bundles oriented parallel to the wound surface, and spindle-shaped fibroblasts and fibrocytes were between them. In this group the maximum number of fibroblasts was observed at this day of the study, in the other experimental groups the fibroblast number was much less (Table 1). The number of vessels in all groups of animals (excepting the control group) decreased (Table 2), this is explained by increasing the degree of maturity of the regeneration tissue. The vessels of the capillary type were perpendicular to the wound surface.

### Conclusion

The results of morphological assessment of the tissue samples from the wounds in the process of their treatment with dressings containing SkQ1 in various concentrations made it possible to conclude that at early steps of treatment (1 to 2 days) the wound coating with minimum content of SkQ1 proved to be the most effective, and at the following steps of the investigation the wound coatings with the higher concentration of SkQ1 were the most effective. At early steps minimum doses of SkQ1 boneficially effect on proliferation of fibroblasts, lead to formation of myofibroblasts in the granulation tissue, stimulate angiogenesis that makes it possible to shorten the phase of inflammation and decrease duration of healing of the experimental wounds. At the later steps (7 days) the best effect (accumulation of fibroblasts, angiogenesis) is observed at maximum concentrations of SkQ1. A change of dressings with various content of SkQ1 at various steps of wound healing may prove to be useful in practical applications.

## Claims

1. A composition comprising an effective amount of a compound of formula (I) for targeted delivery of a biologically active substance into mitohondria to accelerate healing of wounds and other surface damages: 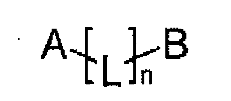 wherein A is an effector group which is an antioxidant 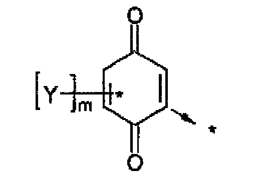 and/or a reduced form thereof
wherein *m* is an integer from 1 to 3; each Y represent the same or different substituents which are a lower alkyl or a lower alkoxy, or two vicinal Y are interconnected to form the structure: 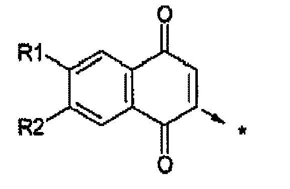 and/or a reduced form thereof
wherein R1 and R2 are the same or different and independently represent a lower alkyl or a lower alkoxy;
L is a linker group which is:
a) a straight or branched hydrocarbon chain optionally substituted with one or more substituents and optionally containing one or more double or triple bonds if necessary;
b) a naturally occurring isoprene chain;
n is an integer from 1 to 20;
B is a) a Skulachev ion Sk:
Sk⁺Z⁻
wherein Sk is a lipophilic cation;
Z is a pharmacologically acceptable anion;
b) a charged hydrophobic peptide containing 1-20 amino acids;
with the proviso that in the compound of structure (I) A is neither ubiquinone (e.g. 2- methyl-4,5-dimethoxy-3,6-dioxo-1,4-cyclohexadiemyl) nor tocopherol or mimetic of superoxide dismutase or ebselen; wherein L is a divalent decyl or divalent pentyl or divalent propyl radical; and B is triphenylphosphonium;
as well as solvates, isomers, pharmaceutically acceptable salts, and a pharmaceutically acceptable carrier thereof.

2. The composition as claimed in claim 1, wherein A is a plastoquinone moiety of the formula: 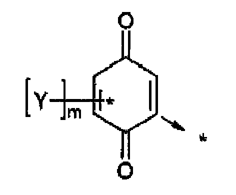 wherein Y is methyl, m is 2,
L is a linker group which is:
a) a straight or branched hydrocarbon chain optionally substituted with one or more substituents and optionally containing one or more double or triple bonds if necessary;
b) a naturally occurring isoprene chain;
n is an integer from 1 to 20;
B is a) a Skulachev ion Sk:
Sk⁺ Z⁻
wherein Sk is a lipophilic cation;
Z is an acceptable anion;
b) a charged hydrophobic peptide containing 1-20 amino acids;
with the proviso that in the compound of structure (1) A is neither ubiquinone (e.g. 2-methyl-4,5-dimethoxy-3,6-dioxo-1,4-cyclohexadienyl) nor tocopherol or mimetic of superoxide dismutase or ebselen; wherein L is a divalent decyl or divalent pentyl or divalent propyl radical; and B is triphenylphosphonium;
as well as solvates, isomers, pharmaceutically acceptable salts thereof, and a pharmaceutically acceptable carrier.

3. The composition as claimed in claims 1, wherein the compound of formula (I) is SkQ1:

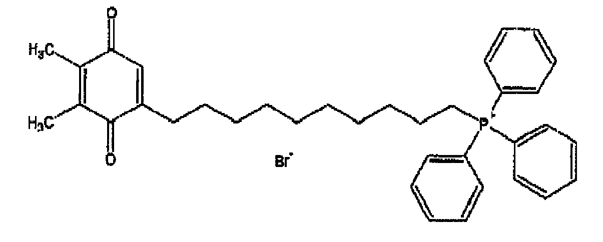

4. Use of the composition as claimed in claims 1 to 3 as a drug overcoming consequences of bums and promoting healing of wounds and surgical sutures.

5. The use as claimed in claim 4, wherein the said drug comprises the compound of general formula (I) in the amount from about 1.0 picogram to about 300 mg/g.

6. The use as claimed in claim 4, wherein the said drug comprises SkQ1 in the amount from about 62 picograms to about 185 mg/g of the drug.

7. The use of the composition as claimed in claims 1 to 3 as a drug for promoting healing of ulcers including trophic ulcers including diabetic wounds and ulcers.

8. The use as claimed in claim 7, wherein the said drug comprises the compound of general formula (I) in the amount from about 1,0 picogram to about 300 mg/g.

9. The use as claimed in claim 7, wherein the said drug comprises SkQ1 in the amount from about 62 picograms to about 185 mg/g.

10. The use of the composition as claimed in claims 1 to 3 as an anti-inflammatory drug.

11. The use of the composition as claimed in claim 10, wherein the said drug comprises a compound of general formula (I) in the amount from about 1.0 pictogram to about 300 mg/g.

12. The use of the composition as claimed in claim 10, wherein the said drug comprises SkQ1 in the amount from about 62 picograms to about 185 mg/g.

13. The use of the composition as claimed in claims 1 to 3 as a drug for protecting healthy tissues from damages during surgical operations.

14. The use as claimed in claim 13, wherein the said drug comprises a compound of general formula (I) in the amount from about 1.0 picogram to about 300 mg/g.

15. The use as claimed in claim 13, wherein the said drug comprises SkQ1 in the amount from about 62 picograms to about 185 mg/g.

16. The use or the composition as claimed in claims 1 to 3 as a drug inhibiting the tissue rejection and preserves the transplantation material.

17. The use as claimed in claim 16, wherein the said drug comprises a compound of general formula (I) in the amount from about 1.0 picogram to about 300 mg/g.

18. The use as claimed in claim 16, wherein the said drug comprises SkQ1 in the amount from about 62 picograms to about 185 mg/g.

19. The use of the composition as claimed in claims 1 to 3 as part of ophthalmic devices, surgical devices, audiologic devices, medical equipment and medical products.

20. The use as claimed in claim 19, wherein the medical products are bandages, films, dressings or plasters.

21. The use of the composition as claimed in claims 1 to 3 in cosmetology including for improving state of the skin, skin rejuvenation, prophylactics or therapy of skin age-related changes including chronological aging, photo aging, skin damages induced by sunlight or ultraviolet radiation.

22. A process for preparing a cosmetic composition comprising a step of introducing a composition according to claims 1 to 3, into the cosmetic composition.
